# EUROPEAN PATENT SPECIFICATION

(11) **EP 1 935 442 B1**
(45) Date of publication and mention of the grant of the patent: **04.08.2010**
(21) Application number: 08004367.2
(22) Date of filing: 01.10.2004
(51) Int. Cl.: A61M 5/142, A61K 33/40, A61B 17/34, A61B 17/56

(54) **Implantable apparatus for administering a therapeutic agent**
Implantierbare Vorrichtung zur Verabreichung eines therapeutischen Mittels
Appareil implantable et procédé pour administrer un agent thérapeutique

(30) Priority: 06.10.2003 US 508300; 15.06.2004 US 867215
(43) Date of publication of application: 25.06.2008
(62) Divisional of application: 04791785.1
(73) Proprietor: Activeo, LLC, Salt Lake City, UT 84095 (US)
(72) Inventor: Murphy, Kieran P. Dr., Baltimore, MD 21210 (US); Muto, Mario, I-80122 Napoli (IT)
(74) Representative: Pawlyn, Anthony Neil

(56) References cited:
- WO-A-01/50983
- WO-A-02/076533
- US-A- 4 193 397
- US-A1- 2002 188 323

## Description

### FIELD OF THE INVENTION

The present invention relates generally to an apparatus for administering a therapeutic agent into tissue and in particular, for the administration of a therapeutic agent such as medical ozone.

### BACKGROUND OF THE INVENTION

Back joint disc or tendon pain is a common and potentially debilitating ailment that affects an estimated 80% of the worldwide population at least once in a lifetime. In many instances, the cause of the pain can be attributed to a degenerated intervertebral disc that has further deteriorated into a condition known as disc herniation This occurs when the disc nucleus pulposus extrudes through a tear or fissure in the outer lining of the disk, thereby exerting pressure on spinal nerves. The compression caused by the herniated nucleus leads to inflammation and is directly responsible for the pain felt down the leg (also referred to as sciatica). Available treatments for this type of back pain vary according to the severity of the hernia. If mild, the patient's condition can be appeased with rest and inactivity for an extended period of time. However, for patients suffering from a severe herniation or who do not respond to non-invasive treatment (pharmacological and/or physical therapy), surgical intervention is often recommended, With this invasive treatment come several disadvantages such as:
i) irreversibility of the procedure
ii) formation of scar tissue
iii) slower recovery time
iv) longer hospital stays

Since the late 1950s, many attempts have been made to treat sciatica and lower back pain with percutaneous procedures to avoid surgery. Well known treatments for example are percutaneous discetomy and chemonucleolysis but the cost of these procedures has kept researchers looking for another alternative. It was in 1984 that an Italian orthopedic surgeon by the name of Dr. Cesare Verga first proposed the use of ozone/oxygen mixtures to treat the pathology of a herniated disk. (See for example, http://www.cleanairassociationlcom/6/ca_3.htm, Ozone Therapy: New breakthrough for Back Treatment, by Gaetano Morello, M.D., the contents of which are incorporated herein by reference.)

Other prior art references include: Percutaneous Treatment of Herniated Lumbar Disc by Intradiscal Oxygen-Ozone Injection, M. Muto and F. Avella, Interventional Neuroradiology 4:279-286, 1998.

In other situations such as rheumatoid arthritis, osteoarthritis or a repetitive injury through sports or occupation, such as tennis elbow, frozen shoulder, or house maids knee, inflammation can develop between two surfaces which are involved in allowing joint function, such as a tendon and the sheath or lubricated tube in which that tendon moves. Inflammation such as bursitis in the knee shoulder hip, or other anatomic bursa may benefit from ozone therapy, this includes epicondylitis, and other tendonitis and bursitis, including the wrist, hand and the tendon sheaths of the hand and wrist. Inflammation can occur at a site where a tendon or a ligament insert to bone or pass through a sheath from trauma, tension, over use or disease.

Inflammation can develop through pathologies of any joint, and these may again include the inflammatory arthropatic conditions of rheumatoid arthritis, psoriatic arthritis and the like, or osteoarthritis. Joints that may be involved in these processes that are amenable to ozone injection include the synovial joints such as the, temperomandibular joint, the hip joint, knee joint, ankle joint, elbow joint or sacro-iliac joint. Vertebral facet and sacro-iliac joints may also benefit, inflammatory involvement of joints in the hand, wrist and feet with rheumatoid arthritis, osteoarthritis or a repetitive injury through sports or occupational such as carpal tunnel syndrome.

The inflammatory and arthritic or degenerative discussions described above are usually treated with a combination of anti-inflammatory agents such as ibuprofen, or more powerful drugs such as steroids or chemotherapy such as methotrexate. It is a common medical practice to inject steroid medications or lidocaine directly into the inflamed tissue or joint. This is often done repeatedly. These drugs can be associated with side effects of infection and even death from gastric ulcer bleeding or immunosurpression and infection. We believe that ozone therapy whether as a liquid or a gas would have advantages over the current practice.

Lavage of a surgical space prior to placement of a permanent surgical implant such as a hip or knee prosthesis, or pacemaker or treatment of an infected joint can be facilitated by the use of medical ozone as a sterilizing substance. Similarly a colostomy stoma can be created such that the adhesive disk is infused with a ozone as a liquid or gas to aid in healing and inhibit infection. The post surgical recovery from sternotomy after cardiac surgery is often complicated wound infection. Placement of a resorbable catheter in the wound that could be irrigated with ozone liquid or gas would aid healing. Indeed any wound could have a resorbable multisided hole catheter placed in it to allow ozone be injected through it. This would have anti-infective, analgesic and promote wound healing properties. This would shorten recovery time and decrease complication rates after surgery.

Enhance liquid ozone could be applied to the wound /surgical site healing at a site of high probability of infection such an abdominal incision/wound after appendectomy, or urgent colectomy with colostomy or after percutaneous endoscopic cholecystectomy.

Endoscopic procedural infusion of ozone and trans catheter infusion of ozone can be used to inhibit the complications endoscopic medical intervention or image guided or non-image guided catheter based intervention for example in endoscopic evaluation of the pancreatic duct.

Dental injection of liquid or gas ozone may augment the preparation and repair of dental cavities, and aid in reduction of root canal inflammation or periodontal disease.

There are veterinary applications of minimally invasive ozone administration in animals diseased with disc and degenerative syndromes. Few other options are available in that arena. Some animals are destroyed due to debilitating pain secondary to pain from disc disease, and arthritis.

While the full therapeutic potential of ozone continues to unfold with ongoing research, it is already clear that this form of therapy for the treatment of disc herniation has significant advantages over other surgical and percutaneous procedures. Some of these advantages include:
- fewer clinical and neuroradiological contraindications
- success rates greater than about 70% in the intervertebral disc
- little or no recovery time
- little or no side effects
- little or no scar tissue formed
- minimally invasive procedure in
- effective alternative treatment for which response to conservative management, such as rest and reduced daily activity, has failed to treat

As the success of ozone gas therapy continues to gain recognition in the medical arena as a non-invasive alternative for the treatment of disc herniation, current methods of administering an effective dose of the ozone are solely as a gas and are far from optimum. There also lacks a sterile methodology through which the ozone can be delivered selectively to the pain-affected area, i.e. the herniated disk. The gas is unstable with a half life measured in seconds. There are no dedicated medical ozone generators that are disposable single use units. In accordance with this, there is a need for equipment especially designed for the treatment of disc herniation and other medical conditions affecting the body with medical ozone so that it can be done in an efficient and sterile manner. There is a need to develop kits for intervention in inflammatory and degenerative disease, that are disposable, or reusable, but aid in creating sterile, stable, ozone rapidly on demand. The generation of ozone liquid from sterile water would allow storage of injector/generators in all medical dental and veterinary facilities.

US 4193397 provides an implantable infusion apparatus including two reservoirs which receive different infusates. The infusates are mixed in a chamber and then dispensed to the patient. The infusate is stored in a the reservoirs in the use format and has to be periodically refilled.

WO02/076533 provides a subcutaneously implanted device for infusion of a drug stored in a tank within that device. Again the therapeutic agent is stored in the format in which it is used.

### SUMMARY OF THE INTENTION

It is therefore an object of the present invention to provide a novel apparatus for administering a gas into a tissue that obviates or mitigates at least one of the disadvantages of the prior art.

In the aspect of the invention there is provided an implantable apparatus for self-contained generation and administration of a therapeutic agent comprising: a chamber for holding the agent; a catheter for connecting the chamber to an area for administration of the agent; a power supply; a therapeutic agent generator in communication with the chamber, the generator connected to the power supply; a switch activateable externally by a patient implanted with the apparatus; the apparatus connected to the power supply such that when the switch is activated the therapeutic agent generator generates the therapeutic agent and fills chamber therewith.

Any suitable source of ozone can be used such as an ozone generator, the AOS-1M Medical Ozonator or AOS-IMS Stainless Medical Ozonator for example, or a disposable injector filled with ozone.

The apparatus may be used to provide a method for the treatment of pain caused by a herniated disc and comprising of first identifying the herniated disk using an imaging technique and subsequently injecting a known volume of medical ozone into the disc and paraspinous soft tissues in a sterile manner and environment.

The therapeutic agent can be gaseous or liquid ozone. The generator can be a medical ozone generator. The medical ozone can be a ratio of oxygen (02) and ozone (03).

The ratio of oxygen to ozone can be about 1 µg/ml, or about 10, µg/ml, or about 20 µg/ml, or about 30 µg/ml, or about 40µg/ml, or about 50µg/ml.

The ratio of oxygen to ozone can be between about 1 µg/ml and about 90 µg/ml. The ratio of oxygen to ozone can be between about 10 µg/ml and about 80 µg/ml. The ratio of oxygen to ozone can be between about 20 µg/ml and about 70µg/ml. The ratio of oxygen to ozone can be between about 10 µg/ml and about 34 µg/ml. More preferably, The ratio of oxygen to ozone can be between about 27 µg/ml to about 28 µg/ml.

The apparatus may be used in a method of treating the pain caused by a herniated disc comprising identifying the herniated disk and injecting medical ozone into the disc and paraspinous soft tissues.

The apparatus may further provide that it includes a syringe for self-contained generation and administration of a therapeutic agent comprising a barrel for holding the agent, and a plunger for insertion into a first end of the barrel and expressing the agent from a second end of the barrel. The syringe also includes a power supply integrally mounted coaxially with the plunger. The syringe also includes a therapeutic agent generator integrally mounted coaxially with the plunger and in communication with the barrel through a channel in the plunger. The generator is connected to the power supply and mounted coaxially with the plunger. The syringe also includes a switch connected to the power supply such that when the switch is activated the therapeutic agent generator generates the therapeutic agent and fills the barrel therewith.

The apparatus may further provide that the ozone can be generated either as a liquid or a gas, in a syringe type structure where the electronics are housed in the area normally occupied by the plunger of the syringe and the anode is in the syringe. The syringes can be any desired volumes, such as those ranging from 1 cc to 60 cc, more preferably 1 cc to 10 ccs. The syringes are typically made of polyethylene to resist the corrosive effect of ozone even in the short time it in contact with the plastic.

### BRIEF DESCRIPTION OF THE DRAWING

Preferred embodiments of the invention will now be discussed, by way of example only, with reference to the attached Figures, in which:
Figure 1 shows an apparatus for administering a gas into a tissue which is used for understanding the embodiments of the invention;
Figure 2 shows the hip area of a patient where a therapeutic agent can be administered using the illustrated administrator or using embodiments of the invention;
Figure 3 shows the spinal disc area of a patient where a therapeutic agent can be administered using the illustrated administrator or using embodiments of the invention;
Figure 4 shows a normal spinal disc;
Figure 5 shows a herniated spinal disc where a therapeutic agent can be administered;
Figure 6 shows an apparatus for administering a gas into a tissue which is used for understanding the embodiments of the invention;
Figure 7 shows the administrator of Figure 6 with the needle placed thereon;
Figure 8 shows an apparatus for administering a gas into a tissue which is used for understanding the embodiments of the invention;
Figure 9 shows an apparatus for administering a therapeutic agent into a hip region in accordance with an embodiment of the invention;
Figure 10 shows the apparatus of Figure 9 in greater detail;
Figure 11 shows an infusion wire through which a therapeutic agent can be administered which is useful as background to the embodiments of the invention;
Figure 12 shows the infusion wire being passed through a needle towards the centre of a herniated disc which is useful as background to the embodiments of the invention;
Figure 13 shows the-infusion wire being placed in centre of a herniated- disc with the needle having been removed which is useful as background to the embodiments of the invention;
Figure 14 shows the infusion wire of Figure 11 in greater detail; and,
Figure 15 shows an apparatus for administering a therapeutic agent into a tissue which is used for understanding the embodiments of the invention;

### DETAILED DESCRIPTION OF THE INVENTION

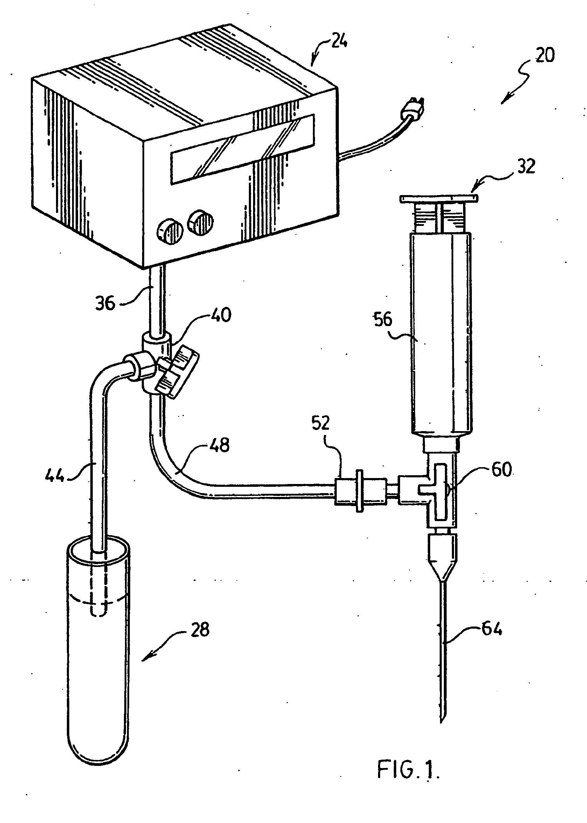

Referring now to Figure 1, an apparatus for administering a therapeutic agent, such as an ozone gas, into a tissue (which is useful for understanding the embodiments of the invention) is indicated generally at 20. In a present embodiment, apparatus 20 comprises an ozone generator 24 that connects to a charcoal scavenger 28 and an ozone administrator 32. Generator 24 can be based on any known medical ozone generator. Generator 24 connects to scavenger 28 and administrator 32 via a network of flexible tubing and valves for selectively directing the flow of gas therebetween. More specifically, a first segment of tubing 36 connects generator 24 to a three-way valve 40. A second segment of tubing 44 connects valve 40 to scavenger 28. A third segment of tubing 48 connects valve 40 to another valve 52, which in turn connects to administrator 32. The tubing is made of any suitable material, such as silicon of the known medical type, and has a diameter and wall thickness to withstand the pressure of ozone gas being carried therethrough. The valves, also known as stopcocks, are also of the known medical type and have fittings complementary to the various portions of tubing.

Administrator 32 is comprised of a syringe 56, a three-way valve 60, and a needle 64. Syringe 56 is typically made of polyethylene to resist the corrosive effect of ozone, but other materials will occur to those of skill in the art. Three-way valve 60 is releasably connectable directly to valve 52, providing a selective pathway between syringe 56 and generator 24 and/or scavenger 28.

Valve 40 has a first position wherein generator 24 communicates only with tubing 48. Valve 52 has a first position wherein tubing 48 communicates with administrator 32. Valve 60 has a first position wherein syringe 56 communicates only with tubing 48. Thus, when valves 40,52 and 60 are all in the first position, generator 24 is in communication with syringe 56, and thus when generator 24 is"on", syringe 56 will be filled with ozone. (It is to be noted that in all of the Figures attached hereto, the various valves depicted therein are not intended to be shown in any specific position, and are merely illustrated to show their physical orientation in relation to the rest of the components in the apparatus.)

Valve 40 has a second position wherein tubing 44 communicates with both tubing 36 and 48. Valve 52 has a second position wherein tubing 48 is effectively capped, preventing communication between administrator 32 and tubing 48. When valves 40 and 52 are in the second position, any ozone generated by generator 24 is captured by scavenger 28. In a present example, scavenger 28 is of the charcoal type, but any type of scavenger for capturing excess ozone can be used.

Once generator 24 is turned "off' after filling syringe 56 as described above, then when valves 40 and 52 are each moved from their respective first position to their respective second position then any excess ozone still present in tubing 48 and 36 will be captured by scavenger 28 and thereby reduce and/or substantially eliminate the unwanted escape of ozone into the atmosphere where it may harm the operator or other individuals proximal to apparatus 20.

Valve 60 also has a second position wherein syringe 56 is prevented from communicating with either valve 52 (or the open fitting on valve 60 that connects to valve 52), or with needle 64. Thus, once syringe 56 has been filled with ozone, leaving administrator 32 'charged' with ozone, valve 60 will also be placed in its second position to retain the ozone within syringe 56 once administrator 32 is disconnected from valve 52.

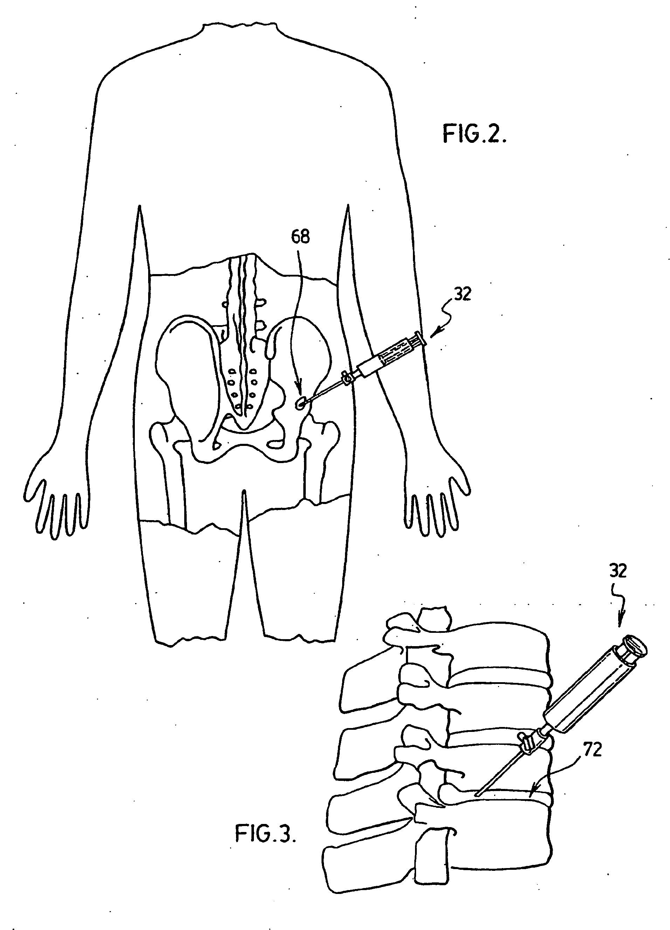

Once administrator 32 is charged with ozone, it can be disconnected from the remainder of apparatus 20 so that it can be used to administer ozone to a target area. Thus, valve 60 also has a third position that places syringe 56 in communication with needle 64. Referring now to Figure 2, when charged, valve 60 can be placed in the second position to prevent ozone from escaping from syringe 56. Administrator 32 can then be disconnected from the remainder of apparatus 20 and then needle 64 can be inserted into tissue (or other target area) where the ozone is to be administered. In Figure 2, needle 64 is shown inserted into a hip 68. Having inserted needle 64 into hip 68, valve 60 is placed in the third position, allowing syringe 56 to communicate with needle 64. At this point syringe 56 is depressed, and the ozone gas therein is expressed out of needle 64 into hip tissue, thereby providing localized pain relief around hip 68. Such administration of ozone can be helpful to relieve pain after some types of hip surgeries, such as hip replacement, or after bone is harvested from the pelvis to use as a bone graft material.

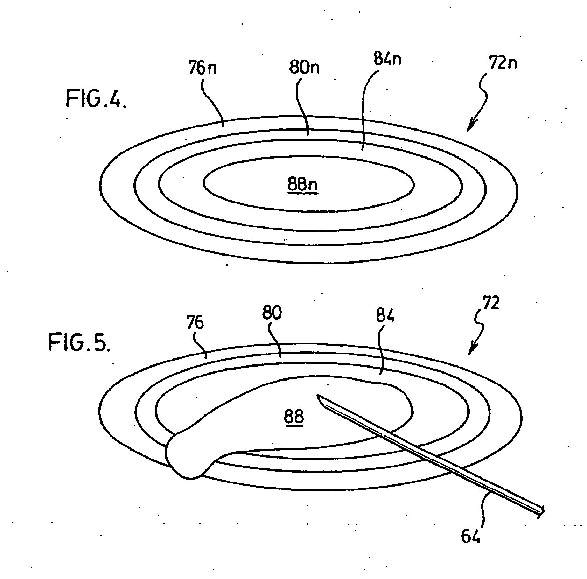

Other types of localized pain relief can also be provided. While Figure 2 depicts pain relief being provided to hip 68, Figure 3 depicts the provision of pain relief to a spinal disc 72. By way of background, as seen in Figure 4 a normal disc is shown in cross section at 72n. Disc 72n has an outer annulus fibrosus 76n, an inner annulus fibrosus 80n, a transition zone 84n, and a nucleus pulposus 88n. However, in Figure 5, disk 72 is also shown in cross section, wherein nucleus pulposus 88 is protruding, and thereby a cause of pain. However, the administration of ozone into the protruding nucleus pulposus 88 using administrator 32 via needle 64 pain can be relieved and/or substantially eliminated at least temporarily.

It should now be apparent that having administered ozone from administrator 32, administrator 32 can then be reconnected to the remainder of apparatus 20, and the appropriate valves 40,50 and 62 adjusted to either allow any remaining ozone to be expressed from administrator 32 for capture by scavenger 28, or to refill syringe 56.

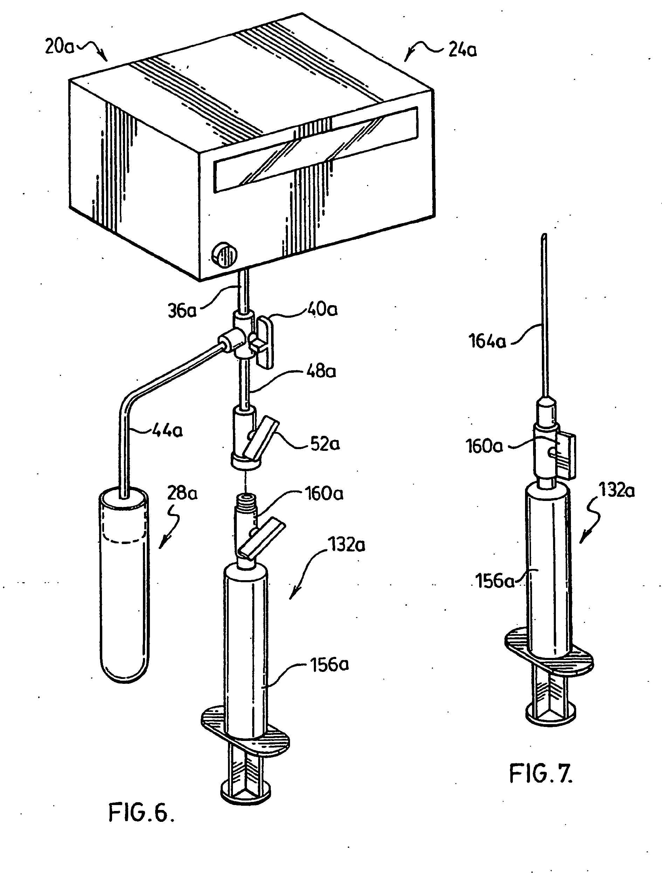

Referring now to Figure 6, an apparatus for administering a gas into a tissue (which is useful for understanding the embodiments of the invention) is indicated generally at 20a, Apparatus 20a includes many similar components to apparatus 20, and like components are indicated with like references but followed by the suffix"a". In contrast to apparatus 20, however, administrator 132a includes certain differences from administrator 32. Specifically, administrator 132a has a two way valve 160a, one end of which connects to syringe 132a, the other end of which has a fitting to allow administrator 132a to be connected to either valve 52a, as shown in Figure 6, or to needle 164a as shown in Figure 7. When administrator 132a is connected to valve 52a as shown in Figure 6, valves 52a and 160a can be placed in an open position so that syringe 132a communicates with generator 24a. When generator 24a is"on", syringe 132a will be filled with ozone. Once syringe 132a is filled with ozone, valves 52a and 160a can be placed in the closed position, and valve 40a can be turned so that once apparatus 160a is disconnected from valve 52a, any ozone remaining in tubes 48a, 36a or elsewhere in that remaining portion of apparatus 24a can be captured by scavenger 28a.

Having filled syringe 132a with ozone, and closed valve 160a, needle 164a can then be affixed thereto as shown in Figure 7. Valve 160a can then be selectively opened or closed to allow the administration of ozone to tissue, in much the same manner as previously described in relation to Figures 2 and 3.

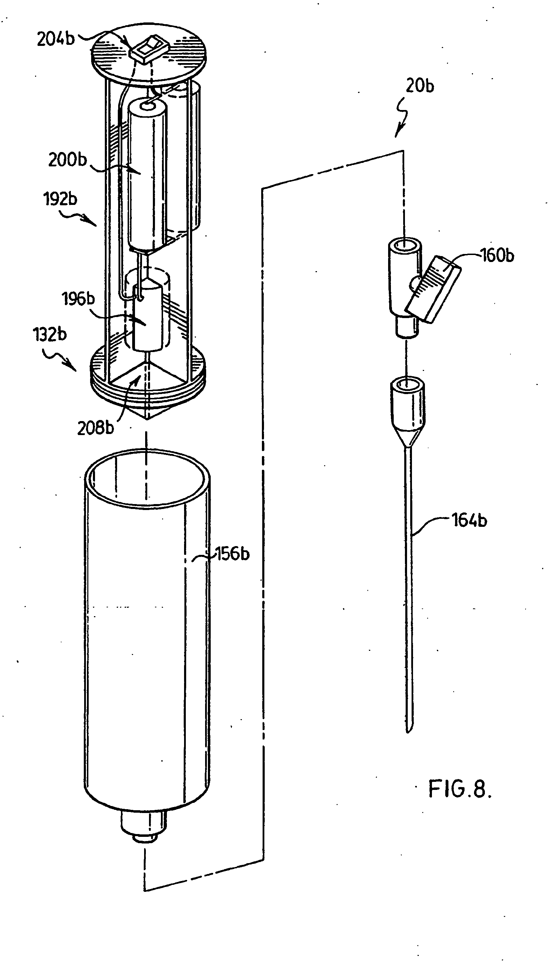

Referring now to Figure 8, an apparatus for administering a therapeutic agent into tissue is indicated generally at 20b. Apparatus 20b is a self-contained, portable version of apparatuses 20 and 20a. Specifically, apparatus 20b includes a needle 164b and a two-way valve 160b that are substantially the same as needle 164a and valve 160a as described above. Apparatus 20b also includes a syringe 132b comprising a barrel 156b (or other chamber) and a plunger 192b (or other means to express the contents of the chamber). Plunger 192b is configured as a normal plunger on a syringe, but also includes an miniature ozone generator 196b mounted on the shaft of plunger 192b. In turn, ozone generator 196b is connected to a power supply 200b and an on-off switch 204b that is disposed on the exterior tip of the shaft of plunger 192b. A small channel 208b joins generator 196b to the opposite tip of the shaft of plunger 192b, such that when plunger 192b is disposed in barrel 156b, generator 196b is in communication with the interior of barrel 156b. In this manner, switch 204b can be activated and thereby cause ozone to be generated and fill barrel 156b. Of particular note, is that the ozone can be generated in either gaseous or liquid form.

While switch 204b is activated, it is generally desired to have valve 160b placed in the closed position to prevent ozone from flowing from barrel 156b into needle 164b. Once a sufficient or otherwise desired amount of ozone has been generated and filled barrel 156b, switch 204b is turned "off" to discontinue generation of ozone, and then apparatus 20b is used in much the same manner as administrator 32 as described above in relation to Figures 2 and 3. The ozone generator 196b can be provided in a variety of sizes, capable of delivering a range of ozone volumes, for example from about one cc to about five cc, but could be made to generate volumes of ozone liquid or gas form of about 0.1 cc to about one litre.

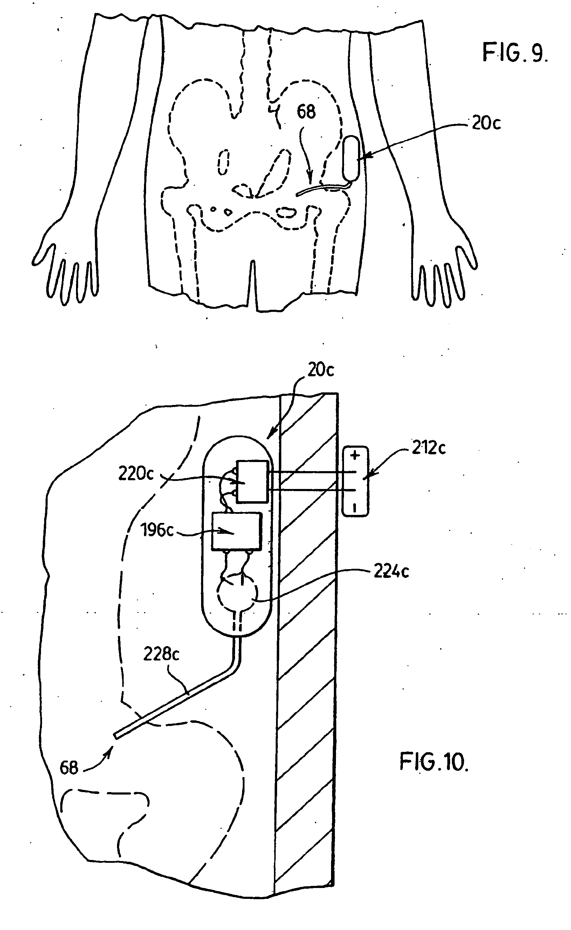

Referring now to Figures 9 and 10, an apparatus according to the invention is provided for administering a therapeutic agent into tissue and is indicated generally at 20c. Apparatus 20c is a self- contained, implantable version of apparatuses 20,20a and 20b. As shown in Figure 9, apparatus 20c is implanted subcutaneously proximal to hip 68. In a present embodiment, apparatus 20c includes a separate, external switchable power supply 212c that is disposed just above the skin of the patient and is thereby activatable on demand by the patient. Power supply 212c connects to a miniature ozone generator 196c disposed percutaneously. In turn, generator 196c is connected to an oxygen source 220c, such that when power supply 212c is "on", generator 196c will interact with source 220c of either oxygen or sterile water, to generate ozone in either in liquid or gaseous form, inside a cavity 224c. In turn, cavity 224c is connected to a catheter 228c, which interconnects with cavity 224c with the tissue inside hip 68 to which the ozone is being administered to relieve pain associated therewith.

As a variation of apparatus 20c, power supply 208c can be disposed subcutaneously, and a wireless switching means can be disposed on the outside of the patient, such as a magnetic switch or other types of wireless means for activating or deactivating the ozone generator. Advantageous to this technique is that the overall use of needles is diminished, and thus beam hardening artifacts that are generated by metallic objects that can prevent proper visualization of the disc details (as might occur when doing injections by means of image guidance, and as may be done in certain other embodiments herein) are reduced. In sum, by reducing the use of needles, visualization is improved and allows the dorsal root ganglion to be clearly seen.

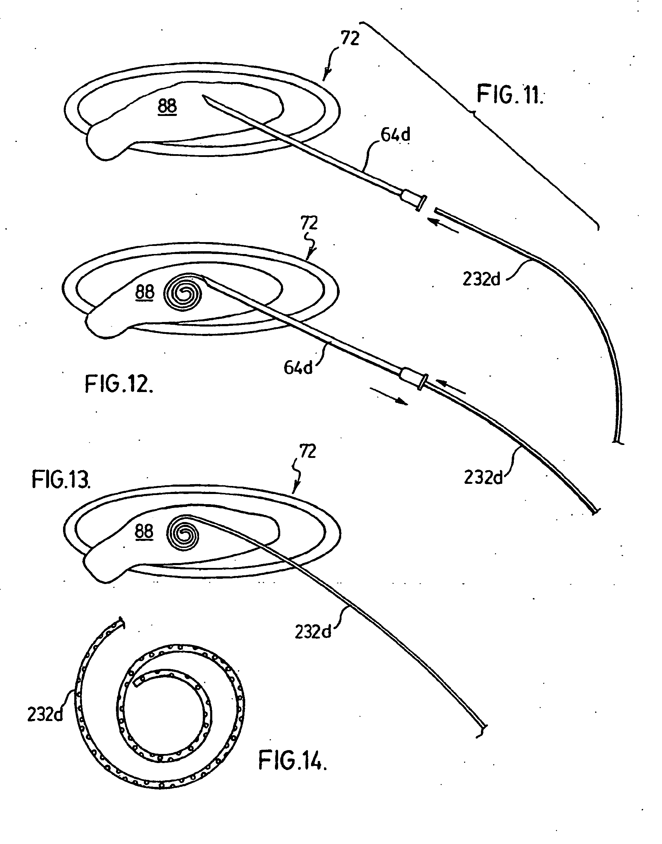

Referring now to Figures 11-14, in another variation (which is useful for understanding the embodiments of the invention) an infusion wire 232d is passed through needle 64d until it coils inside nucleus pulposus 88 (or other tissue area for treatment.) Since infusion wire 232d is perforated along its length, as shown in Figure 14, once a desired or sufficient amount of wire 232d has been inserted into nucleus pulposus 88, an ozone source can be connected to the opposite end of the infusion wire 232d and injected into nucleus pulposus 88 via the infusion wire. Due to the perforations along infusion wire 232d, ozone is dispersed more readily into nucleus pulposus 88.

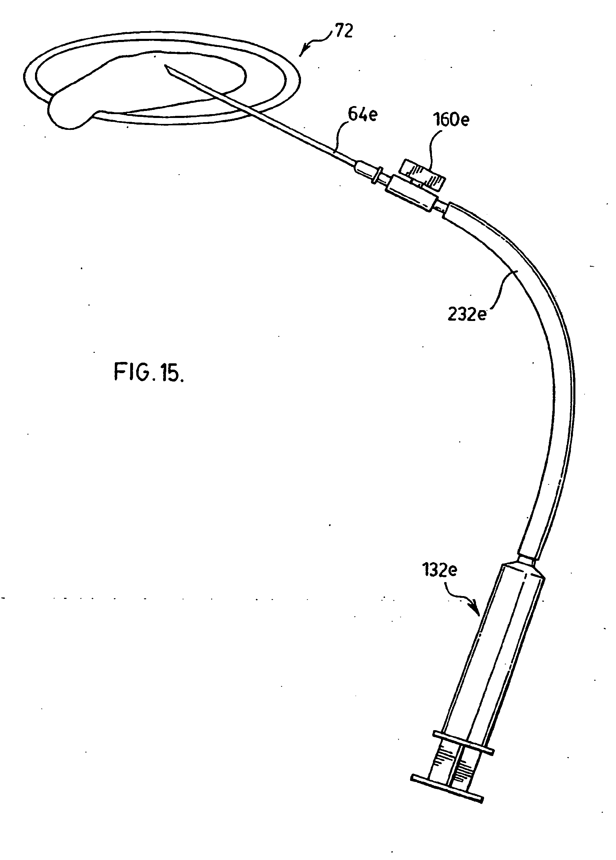

Referring now to Figure 15, in another variation (which is useful for understanding the embodiments of the invention) it can be seen that various other configurations of how administration of the ozone is provided are shown. Specifically, Figure 15 shows a syringe 132e connected to a valve 160e via tubing 232e.

A kit of parts for performing an injection of medical ozone for the treatment of a herniated disc or the like can be provided. The kit includes a sterile tray with a number of compartments to hold :
- a disinfectant, drape and skin preparation material,
- lidocaine and a 10 cc syringe with a 22-G long needle,
- nonionic x-ray dye-for discogram such as Omnipaque 300 M,
- outer 16 or 18 G needle to act as the co-axial introducing needle,
- 20 or 22 G needle with possible side holes for injecting the ozone into the disc,
- outer large needle of 16-18 G for steroid injection (can also be used to inject ozone into the paraspinous soft tissue),
- an infusion wire for even distribution of the ozone into the disc space
- a charcoal ozone scavenger (a bottle with charcoal and 100 % 02),
- steroids and local anaesthetics contained in a vial, ready for injection,
- a source of ozone such as a generator, examples include the AOS- 1M Medical Ozonator or AOS-IMS Stainless Medical Ozonator, or a disposable injector in which then ozone can be generated filled with ozone,
- a disposable filtered attachment for the syringe a one way stop cock attached to the ozone generator,
- connecting, non-compliant tubing of 20-30 cm in length to reduce radiation to the operator hands during injection under image guidance.
- a three-way stop cock linking the tubing to the charcoal ozone scavenger bottle
- a one-way stop cock linking the tubing to the syringe via the said disposable filtered adapter,
- pharmaceutical gel such as Povidone, and
- adhesive bandages with a gauze pad in the center, such as Band- aid, or small dressing.

A method consisting of first introducing the patient into the computer tomography ("CT") scanner and performing a diagnostic CT scan in order to identify the herniated disc such as disc 72 is also useful. The CT gantry is subsequently draped and readied, the skin is prepared and local anaesthetic is applied to the skin and adjacent soft tissue. An 16-18 G needle is pinned down to the disc level and a 22 G needle is inserted into the disc. A discogram is performed to check symptoms by injection of x-ray dye. The ozone apparatus (in the form of any of the previously described apparatus or such other apparatus as may now occur to those of skill in the art) is subsequently switched on and the oxygen/ozone concentration chosen. The oxygen/ozone mixtures to choose from are in the about 0 µg/ml, about 10 µ/ml, about 20µg/ml, about 30 µg/ml, about 40 µg/ml or about 50µg/ml. A presently preferred range is between about 10 µg/ml and about 34 µg/ml, but more preferably about 27 µg/ml to about 28 µg/ml. In other examples other gases or therapeutic agents can be used, such as pure oxygen if found to be therapeutically effective or desirable.

As a next step, the syringe is fitted with a disposable filtered attachment which is itself attached to 20-30 cm of non-compliant tubing fitted with a 3-way to cock on one end and a one-way stop cock on the other, such as that shown in Figure 15. Ozone gas is then aspirated into the 10-cc syringe (can also use a calibrated cardiac syringe) via the disposable filtered adapter and the connecting tubing such that the entire dead space is filled with a known concentration of ozone. The total volume of dead space should be known so appropriate amounts of ozone are actually injected to the desired area. CT fluoro imaging or the like can be subsequently used to inject the ozone into the disc and paraspinous soft tissues. An infusion wire with a stiff stylet could be used, as previously described. Once the ozone injection is completed, a stop cock on the connecting tubing is closed. All of the leftover ozone is injected into the charcoal scavenger or a pure oxygen tube/bottle attached to the connecting tubing via a Luer lock. Steroids are then injected through the coaxial outer large needle. Finally, all needles are removed, the skin is appropriately dressed and a bandage is used to cover the perforated skin.

The combined intradiscal and periganglionic injection of medical ozone and periganglionic injection of steroids, which has an cumulative effect and enhances the overall outcome of treatment may be provided.

Other types of pain can be treated using the teachings herein. For example, joints, tendons, ligaments are other areas that can be treated. Another example includes irrigating a wound site, such as a colostomy, with ozone to reduce pain at the wound site. As another example, an implantable device could put into the teeth (or other dental area) of a patient, similar to apparatus 20c to reduce pain in the dental region. As another example is to irrigate a subcutaneous pouch for holding a pacemaker or the like for sterilization and/or treatment of pain and/or decrease of inflammation and such other advantage corresponding to the therapeutic agent as will occur to those of skill in the art.

## Claims

1. An implantable apparatus (20c) for self-contained administration of a therapeutic agent comprising:
a chamber (224c) for holding said agent;
a catheter (228c) for connecting said chamber (224c) to an area (68) for administration of said agent;
a power supply (212c);
a switch (212c) activatable externally by a patient implanted with said apparatus (20c);
**characterised in that** the implantable apparatus is for self-contained generation of the therapeutic agent and further comprises:
a therapeutic agent generator (196c) in communication with said chamber (224c), said generator (196c) connected to said power supply (212c); and wherein
said apparatus (20c) is connected to said power supply (212c) such that when said switch (212c) is activated said therapeutic agent generator (196c) generates said therapeutic agent and fills the chamber (224c) therewith.

2. The implantable apparatus (20c) of claim 1 wherein said agent is ozone and said therapeutic agent generator (196c) is an ozone generator having a supply of oxygen integrally available therewith.

3. The implantable apparatus (20c) of claim 1, wherein the therapeutic agent generator (196c) is connected to an oxygen source.

4. The implantable apparatus (20c) of claim 3, wherein the oxygen source is oxygen or sterile water.

5. The implantable apparatus (20c) of claim 1, wherein the power supply (212c) is adapted to be disposed subcutaneously and wireless switching means is adapted to be disposed on the outside of the patient for activating or deactivating the therapeutic agent generator (196c), the therapeutic agent generator (196c) being an ozone generator.

6. The implantable apparatus (20c) of claim 5, in which the wireless switching means is a magnetic switch.

## Patentansprüche

1. Implantierbare Vorrichtung (20c) zur eigenständigen Verabreichung eines therapeutischen Mittels, umfassend:
eine Kammer (224c) zur Aufnahme des Mittels;
einen Katheter (228c) zur Verbindung der Kammer (224c) zu einem Bereich (68) zur Verabreichung des Mittels;
einer Stromversorgung (212c);
einen Schalter (212c), der extern durch einen Patienten aktivierbar ist, welchem die Vorrichtung (20c) implantiert ist;
**dadurch charakterisiert, dass** die implantierbare Vorrichtung zur eigenständigen Erzeugung des therapeutischen Mittels ist und des weiteren umfasst:
einen Therapeutisches-Mittel-Generator (196c) in Kommunikation mit der Kammer (224c), wobei der Generator (196c) mit der Stromversorgung (212c) verbunden ist, und wobei
die Vorrichtung (20c) mit der Stromversorgung (212c) dergestalt verbunden ist, dass wenn der Schalter (212c) aktiviert wird, der Therapeutisches-Mittel-Generator (196c) das therapeutische Mittel erzeugt und die Kammer (224c) damit füllt.

2. Implantierbare Vorrichtung (20c) gemäß Anspruch 1, wobei das Mittel Ozon ist und der Therapeutisches-Mittel-Generator (196c) einen Ozongenerator mit einer Zufuhr von Sauerstoff, welche ganzheitlich damit verfügbar ist, darstellt.

3. Implantierbare Vorrichtung (20c) gemäß Anspruch 1, wobei der Therapeutisches-Mittel-Generator (196c) mit einer Sauerstoffquelle verbunden ist.

4. Implantierbare Vorrichtung (20c) gemäß Anspruch 3, wobei die Sauerstoffquelle Sauerstoff oder steriles Wasser ist.

5. Implantierbare Vorrichtung (20c) gemäß Anspruch 1, wobei die Stromversorgung (212c) so angepasst ist, dass sie subkutan angeordnet ist, und eine drahtlose Schaltvorrichtung so angepasst ist, dass sie auf der Außenseite des Patienten angeordnet ist zur Aktivierung oder Deaktivierung des Therapeutisches-Mittel-Generators (196c), wobei der Therapeutisches-Mittel-Generator (196c) ein Ozongenerator ist.

6. Implantierbare Vorrichtung (20c) gemäß Anspruch 5, wobei die drahtlose Schaltvorrichtung ein magnetischer Schalter ist.

## Revendications

1. Appareil implantable (20c) pour une administration auto-contenue d'un agent thérapeutique comprenant :
une chambre (224c) pour contenir ledit agent ;
un cathéter (228c) pour connecter ladite chambre (224c) à une superficie (68) pour l'administration dudit agent ;
une alimentation électrique (212c) ;
un commutateur (212c) pouvant être activé extérieurement par un patient dans lequel on a implanté ledit appareil (20c) ;
**caractérisé en ce que** l'appareil implantable est destiné à la génération auto-contenue de l'agent thérapeutique et comprend en outre :
un générateur d'agent thérapeutique (196c) en communication avec ladite chambre (224c), ledit générateur (196c) connecté à ladite alimentation électrique (212c) et dans lequel
ledit appareil (20c) est connecté à ladite alimentation électrique (212c) de sorte que, lorsque ledit commutateur (212c) est activé, ledit générateur d'agent thérapeutique (196c) génère ledit agent thérapeutique et remplit la chambre (224c) avec lui.

2. Appareil implantable (20c) selon la revendication 1, dans lequel ledit agent est l'ozone et ledit générateur d'agent thérapeutique (196c) est un générateur d'ozone ayant une alimentation d'oxygène intégralement disponible.

3. Appareil implantable (20c) selon la revendication 1, dans lequel le générateur d'agent thérapeutique (196c) est connecté à une source d'oxygène.

4. Appareil implantable (20c) selon la revendication 3, dans lequel la source d'oxygène est de l'oxygène ou de l'eau stérile.

5. Appareil implantable (20c) selon la revendication 1, dans lequel l'alimentation électrique (212c) est adaptée, de façon à être disposée par voie sous-cutanée et des moyens de commutation sans fil sont adaptés pour être disposés sur l'extérieur du patient pour activer ou désactiver le générateur d'agent thérapeutique (196c), le générateur d'agent thérapeutique (196c) étant un générateur d'ozone.

6. Appareil implantable (20c) selon la revendication 5, dans lequel les moyens de commutation sans fil sont un commutateur magnétique.
